# EUROPEAN PATENT SPECIFICATION

(11) **EP 2 371 938 B1**
(45) Date of publication and mention of the grant of the patent: **02.10.2013**
(21) Application number: 09835123.2
(22) Date of filing: 25.12.2009
(51) Int. Cl.: C11D 1/29, C11D 3/04, C11D 3/20, C11D 17/08, A61K 8/34, A61Q 19/10, A61K 8/46

(54) **SURFACTANT COMPOSITION**
TENSIDZUSAMMENSETZUNG
COMPOSITION D'AGENT TENSIOACTIF

(30) Priority: 25.12.2008 JP 2008330495; 16.12.2009 JP 2009284895
(43) Date of publication of application: 05.10.2011
(73) Proprietor: Kao Corporation, Chuo-Ku Tokyo 103-8210 (JP)
(72) Inventor: MITSUDA, Yoshinori, Wakayama-shi Wakayama 640-8580 (JP); TOMIFUJI, Takeshi, Wakayama-shi Wakayama 640-8580 (JP); FUKUSHIMA, Tetsuaki, Wakayama-shi Wakayama 640-8580 (JP)
(74) Representative: HOFFMANN EITLE
(86) International application number: PCT/JP2009/071891
(87) International publication number: WO 2010/074342

(56) References cited:
- WO-A1-2009/008542
- JP-A- 55 084 399
- JP-A- 62 246 549
- JP-A- 2000 345 191
- JP-A- 2000 345 191
- JP-A- 2007 176 894
- JP-A- 2008 156 608
- JP-A- 2008 156 608
- JP-A- 2009 143 980

## Description

### Field of the invention

The present invention relates to a surfactant composition, and particularly to a surfactant composition containing an alkyl ether sulfate ester salt having a specific structure.

### Background of the invention

Polyoxyalkylene alkyl ether sulfate ester salts are anion surfactants produced by sulfating a higher alcohol or a higher alcohol-alkylene oxide adduct. These sulfates are gentle to the skin, and thus widely used as a main active agent in liquid detergents such as dishwashing detergent, shampoo, and detergent for clothes.

Such a polyoxyalkylene alkyl ether sulfate ester salt is distributed to the market in the form of not a single material but a composition mainly composed of the sulfate and water due to the standard method of production thereof. In the composition, a content of the sulfate is generally less than 30% by mass or around 70% by mass according to physical properties of the solution. A composition having a content of the sulfate of less than 30% by mass, however, is not preferred from the viewpoints of volume of a storage and/or reaction tank for the composition, production efficiency, and cost associated with transportation. The content is desirably as high as possible. A composition having a content of the sulfate of more than 80% by mass losses fluidity, which is unfavorable from the viewpoint of handling.

As described above, a composition containing a polyoxyalkylene alkyl ether sulfate ester salt at high concentration is useful at an industrial phase. For producing such a composition, there have been various techniques proposed. JP-A49-15706 discloses a method of production that enables to produce a solution containing ethanol, water, and a higher secondary alcohol ethoxylate sulfate ester salt at a specific ratio. JP-A03-93900 discloses a method of production that enables to produce a liquid surface active composition containing an alcohol ethoxysulfate at high concentration and substantially no lower alcohol such as ethanol and methanol.

Moreover, GB-A1428273 discloses a liquid detergent composition containing an alcohol ethoxysulfate in the form of a concentrated uniform solution by blending a primary alcohol having 2 to 4 carbon atoms or isopropanol.

JP 2000-345191 A relates to an anionic surfactant which consists of a compound of the formula RO-(EO)ₓ-(PO)_{y}-(EO)_{z}-A, wherein R is an 8-24C aliphatic hydrocarbon; EO is oxyethylene; PO is oxypropylene; (x) and (z) are each the mean addition number of moles of ethylene oxide, being 1-50; (y) is the mean addition number of moles of propylene oxide, being 1-50; A is a counterion-containing anion; and (EO)ₓ, (PO)_{y} and (EO)_{z} are in block linkage in this order, e.g. C₁₂H₂₅O-(EO)₂-(PO)₂-(EO)₃-SO₃Na. The surfactant is said to be obtainable, for example, by charging lauryl alcohol and potassium hydroxide into a rotary agitation type autoclave for both ethylene oxide and propylene oxide, followed by temperature rise to effect dehydration, then by charging ethylene oxide into the autoclave to carry out a reaction, followed by charging propylene oxide into the autoclave to carry out a further reaction, and subsequently by neutralizing the reaction product with acetic acid.

JP 2008-156608 A relates to a liquid surfactant composition that contains (a) 40-58 mass% mixture of a compound represented by general formula (I): RO(AO)ₙSO₃M, in the mixture the ratio of a compound, of which R in general formula (1) is a hydrocarbon group having a branched chain, is 15 mass% or higher and not higher than 50 mass%, (b) 10-20 mass% at least one kind of a 16C lower alcohol, and (c) 22-50 mass% water. In general formula (1), R is a 9-24C hydrocarbon group having a linear or branched chain; A is a 2-4C alkylene group; M is an alkali metal or the like; and n is such a number that its mean value in component (a) is 0-100.

### Summary of the invention

The present invention relates to a surfactant composition having a pH (20°C) of 10 to 13.6, containing:
(a) 40 to 58% by mass of a compound represented by formula (1); (b) 13 to 20% by mass of an alcohol having 1 to 6 carbon atoms; (c) 0.002 to 0.5% by mass of an alkaline agent; and water:

   RO- [(PO)ₘ/(EO)ₙ]SO₃M (1)

   wherein, R represents a hydrocarbon group having 8 to 24 carbon atoms; PO and EO represent a propyleneoxy group and an ethyleneoxy group, respectively; m and n represent number average addition mole numbers of PO and EO, respectively, and satisfy 0<m<5 and 0<n≤20, respectively; and M represents a cation (excluding a hydrogen ion); wherein the addition mode of PO and EO may be block or random.

### Detailed description of the invention

A composition containing a polyoxyalkylene alkyl ether sulfate ester salt at a high concentration according to the prior art can deteriorate in odor and/or color under conditions of long-term storage.

The present invention provides a surfactant composition containing a polyoxyalkylene alkyl ether sulfate ester salt having a structure formed by addition of propylene oxide at high concentration, that has good stabilities of odor and color under conditions of long-term storage.

The present invention provides a surfactant composition containing a polyoxyalkylene alkyl ether sulfate ester salt having a structure formed by addition of propylene oxide at a high concentration, that has good stabilities of odor and color under conditions of long-term storage.

The surfactant composition of the present invention is useful for a detergent base material.

### [Component (a)]

In formula (1), R preferably represents a linear or branched, alkyl or alkenyl group, having 8 to 20 carbon atoms. R having 10 to 20 carbon atoms results in a better balance between surface active performance and solubility in water of the polyoxyalkylene alkyl ether sulfate ester salt. Examples of the alkyl group and the alkenyl group include a decyl group, a dodecyl group, a tetradecyl group, a hexadecyl group, an octadecyl group, an icosyl group and an octadecenyl group.

Component (a) preferably contains a compound of formula (1) in which R represents an alkyl group, and more preferably R represents an alkyl group having 8 to 16 carbon atoms, even more preferably 10 to 14 carbon atoms, and still even more preferably 12 to 14 carbon atoms. For component (a), those produced with an alcohol derived from a petrified material or an alcohol derived from natural fat-and-oil can be used.

In formula (1), m represents an addition mole number of PO. An average number of m of component (a) is more than 0 and less than 5. When the composition of the present invention is used as a main active agent of a liquid detergent, considering long-term stability for storage, the average number is preferably 0.1 to 3. Considering basic properties such as detergency, low-temperature stability, physical properties of a detergent composition, and biodegradability in a comprehensive manner, the average number is preferably 0.1 to 3, more preferably 0.2 to 2, and even more preferably 0.3 to 0.9. In formula (1), the "/" is a symbol for separating PO from EO for convenience.

In formula (1), n represents an addition mole number of EO. An average number of n of component (a) is more than 0 and 20 or less. Similarly to the addition mole number of PO, considering basic properties of the composition of the present invention as a main active agent of a liquid detergent in a comprehensive manner, the average number is preferably 0.3 to 12, and more preferably 0.6 to 10. Further considering detergency and stability, the average number is more preferably 0.8 to 5, and even more preferably 1 to 2.3.

An addition mode of PO and EO in formula (1) may be random addition or block addition. In cases of block addition, EO and PO can be arbitrarily added in any order. For example, these are preferably added in orders such as EO/PO, PO/EO, and EO/PO/EO. Among them, preferred are compounds in which RO- is linked to PO and EO in this order by block addition.

In formula (1), M represents a cation group to form a salt. Examples of the cation group include alkaline metal ions, alkaline earth metal ions, ammonium ions, and alkanolammonium ions such as mono-, di-, and triethanolammonium ions. Examples of the alkaline metal include sodium, potassium, and lithium. Examples of the alkaline earth metal include calcium. Among these metals, preferred are sodium and potassium, and more preferred is sodium.

For component (a), among compounds represented by formula (1), preferred is a compound represented by formula (1') in which RO- is linked to PO and EO in this order by block addition. For formula (1'), preferred R, m, and M are same to those for formula (1), respectively.

RO-(PO)ₘ-(EO)ₙSO₃M (1')

wherein, R represents a hydrocarbon group having 8 to 24 carbon atoms; PO and EO represent a propyleneoxy group and an ethyleneoxy group, respectively; m and n represent number average addition mole numbers of PO and EO, respectively; and satisfy 0<m<5 and 0<n≤20, respectively; and M represents a cation (excluding a hydrogen ion).

Component (a) used in the surfactant composition of the present invention can be prepared by any process without specific limitation. For example, when component (a) contains a compound in which PO and EO are added to RO- in this order by block addition, component (a) can be prepared by a process including the following steps (I) to (III).
step (I): adding propylene oxide to 1 mole of an alcohol, having a hydrocarbon group having 8 to 24 carbon atoms, preferably an alkyl group having 8 to 24 carbon atoms, within the range from more than 0 mol to less than 5 moles on the average;
step (II): adding ethylene oxide to the propylene oxide-adduct from step (I) within the range from more than 0 mol to 20 mol or less on the average;
step (III): sulfating the alkoxylate from step (II) and neutralizing it.

Specific examples of the alcohol used in step (I) include natural alcohols derived form coconut oil, palm oil, beef tallow or etc. and synthetic higher alcohols obtained by an oxo process, the Ziegler method, direct oxidation of paraffin or etc. The alcohol may be saturated or unsaturated, and may be either primary or secondary. Preferred are saturated primary alcohols. The alcohol may also be blanched.

A used amount of propylene oxide to 1 mol of the alcohol is preferably such an amount as to produce the polyoxyalkylene alkyl ethersulfate represented by formula (I), and more specifically more than 0 mole to less than 5 moles to 1 mole of the alcohol. When the composition of the present invention is used as a main active agent of a liquid detergent, considering basic properties such as detergency, low-temperature stability, biodegradability, and physical properties of a detergent composition in a comprehensive manner, the amount is preferably 0.1 to 3 mol. From the reactivity in production, the amount is more preferably 0.2 to 2 mol, and more preferably 0.3 to 0.9 mol.

Step (II) is to add ethylene oxide to the propylene oxide-adduct obtained in step (I) within the range from more than 0 mol to 20 mol or less at an average. A used amount of ethylene oxide to 1 mol of the alcohol is preferably such an amount to produce component (a), and more specifically more than 0 mol to 20 mol or less to 1 mol of the alcohol. Considering basic properties as a main active agent of a liquid detergent in a comprehensive manner similarly for the addition mole number of PO, the amount is preferably 0.3 to 12 mol, and more preferably 0.6 to 10 mol. Further considering detergency and stability, the amount is more preferably 0.8 to 5 mol, and even more preferably 1 to 2.3 mol.

For performing these steps (I) and (II), a conventional known method can be employed. Thus, an autoclave is charged by the alcohol and a catalyst such as potassium hydroxide (KOH) in an amount of 0.5 to 1% by mol to the alcohol. They are heated and dehydrated. To this are added propylene oxide and ethylene oxide in given amounts and reacted at 120 to 160°C. In the process, a mode of addition is block addition. The process is performed in the order of addition of propylene oxide (step (I)) and addition of ethylene oxide (step (II)). The autoclave used is preferably equipped with a stirring device, a temperature controller, and an automatically-introducing device.

Step (III) is to sulfate the alkoxylate from step (II) and to neutralize it. For sulfating, the sulfating is conducted with sulfur trioxide (liquid or gas), sulfur trioxide-containing gas, fuming sulfuric acid or chlorosulfonic acid. From the viewpoint of particularly preventing generation of waste sulfuric acid, hydrochloric acid and the like, preferably employed is a continuous supply of sulfur trioxide in the form of gas or liquid together with the alkoxylate.

For neutralizing the sulfated product, those methods can be employed, including a batch method of adding the sulfated product to a neutralizer in a given amount and stirring to neutralize, a continuous method of supplying the sulfated product and a neutralizer into a pipeline without interruption and neutralizing in a stirring-mixer. In the present invention, any method can be employed for neutralizing. Examples of the neutralizer used in this step include aqueous solutions of alkaline metals, ammonia water, and triethanolamine. Preferred are aqueous solutions of alkaline metals, and more preferred is an aqueous solution of sodium hydroxide.

Component (a) thus prepared is preferably used in a form of an aqueous solution at a concentration of 10 to 70% by mass. The aqueous solution is used to obtain given proportions of components (a) to (c) of the present invention. In the present invention, in order to simplify the production, it is preferable to mix the neutralizer (e.g., sodium hydroxide) or an aqueous solution thereof with component (b) and carry out the neutralization. The neutralizer may also be added together with component (c), or with these components (b) and component (c). In the case of using a compound corresponding to component (c) as the neutralizer, the amount of component (c) is a necessary amount to neutralize a sulfate precursor of the compound (a), for example, the sulfate obtained at step (III); then the amount required for component (c) of the surfactant composition of the present invention, 0.002 to 0.5% by mass, preferably 0.025 to 0.3% by mass, and more preferably 0.05 to 0.1% by mass, and further an amount to adjust the surfactant composition to have a pH (20°C) of 10 to 13.6, preferably 11 to 13.5, and more preferably 11.5 to 13.4, in order to obtain the surfactant composition of the present invention simply.

Thus, the present invention provides a method for producing a surfactant composition containing 40 to 58% by mass of component (a), 13 to 20% by mass of component (b), 0.002 to 0.5% by mass of component (c), and water, including the following steps (i) to (iii).
step (i): adding propylene oxide and ethylene oxide to 1 mol of an alcohol having a hydrocarbon group having 8 to 24 carbon atoms, preferably an alkyl group having 8 to 24 carbon atoms, within the range from more than 0 mol to less than 5 mol and from more than 0 mol to 20 mol or less, respectively;
step (ii) : sulfating the alkoxylate obtained at step (i) ; step (iii) : adding a mixture of component (b), component (c) and water to the sulfated alkoxylate obtained at step (ii), in which an amount of component (c) is larger than that required for neutralizing the sulfated alkoxylate, to obtain a mixture containing component (a) resulting from the neutralization of the sulfated alkoxylate, component (b), and component (c).

Step (i) can be performed as described for steps (I) and (II). The sulfation of step (ii) can be performed similarly as described for step (III). Step (iii) uses a liquid mixture (preferably aqueous solution) containing component (b), component (c) and water, in which an amount of component (c) is larger than that required for neutralizing the sulfated alkoxylate, for neutralizing the sulfated alkoxylate and also for providing component (c) in a given amount. For component (c), the "a larger amount than that required for neutralizing the sulfate" can be a theoretical value derived from the starting materials. Component (c) is also used in such amount as that the produced surfactant composition has a final pH (20°C) of 10 to 13.6, preferably 11 to 13.5, and 10 more preferably 11.5 to 13.4. When the resultant mixture obtained at step (iii) contains components (a) to (c) within the ranges of the present invention, it is the surfactant composition of the present invention. It can be used as it is or by changing the composition within the ranges of the present invention. When the resultant mixture contains components (a) to (c) in amounts out of the ranges of the present invention, the amounts are adjusted to be within the ranges of the present invention to obtain a surfactant mixture according to the present invention.

The surfactant composition of the present invention contains a polyoxyalkylene alkyl ether sulfate ester salt represented by formula (1) as component (a) in an amount of 40 to 58% by mass, preferably 50 to 58% by mass, and more preferably 52 to 56% by mass. In the present invention, the "polyoxyalkylene alkyl ether sulfate ester salt" includes those having other groups such as an alkenyl group than the alkyl group for convenience sake.

### [Component (b)]

Component (b) can be any alcohol without specific limitation. Examples of the alcohol include methanol, ethanol, propanol, butanol, pentanol, and hexanol. From the points of generality and ease of handling, ethanol is preferred. The surfactant composition of the present invention contains component (b) in an amount of 13 to 20% by mass, preferably 13 to 18% by mass, and more preferably 13 to 16% by mass.

### [Component (c)]

Component (c) is not specifically limited. Examples of the alkaline agent include inorganic compounds such as sodium hydroxide and potassium hydroxide and organic compounds such as monoethanolamine, diethanolamine, and triethanolamine. Preferred are inorganic compounds such as sodium hydroxide and potassium hydroxide, and more preferred is sodium hydroxide. The surfactant composition of the present invention contains component (c) in an amount of 0.002 to 0.5% by mass, preferably 0.025 to 0.3% by mass, and more preferably 0.05 to 0.1% by mass.

### [Surfactant composition]

The surfactant composition of the present invention may further contain a component other than components (a) to (c) within the range that the composition can keep its stability. The total amount of components (a) to (c) preferably accounts for 55% or more by mass, and more preferably 60 to 99% by mass of the composition.

The surfactant composition of the present invention contains water. A content of water is preferably 45% or less by mass, and more preferably 25 to 40% by mass of the composition. The surfactant composition of the present invention may be an aqueous solution containing components (a) to (c). The composition may further contain an additive such as a pH buffer agent such as phosphate and citrate, a preservative, an antibacterial agent, and a metal chelating agent. Other components, introduced during the process of producing component (a) and other components, for example, a remaining polyoxyalkylene alkyl ether after sulfation and inorganic sulfate salts side-produced in the neutralization, may be present in the surfactant composition of the present invention as long as they do not impair the advantageous effects of the present invention.

The surfactant composition of the present invention preferably has a viscosity of 1000 mPa·s or less, more preferably 500 mPa·s or less, even more preferably 200 mPa·s or less, and still even more preferably 100 mPa·s or less at an ambient temperature (20°C). The viscosity is measured with a Brookfield (B type) viscometer under a condition of 20°C.

From the viewpoint of storage stability for a long period, the surfactant composition of the present invention has weak alkaline to alkaline pH (20°C) ( a pH of 10 to 13.6), preferably 11 to 13.5, and more preferably 11.5 to 13.4. The surfactant composition of the present invention can be preferably used as an ingredient for a liquid detergent composition. In this case, the liquid detergent composition can further contain auxiliaries such as a colorant, a flavorant, a solubilizing agent, and a builder. To control detergency and foaming properties, the liquid detergent composition can further contain other surfactants such as an anionic, a cationic, an amphoteric, and an amide-based nonionic surfactants. The liquid detergent composition can be used in shampoo, body-wash, kitchen detergent, and liquid soap.

### Examples

The following Examples demonstrate the present invention. Examples are intended to illustrate the present invention and not to limit the present invention.

### Examples 1 to 5 and Comparative Examples 1 to 2

For each Examples and Comparative Examples, in an autoclave equipped with a stirring device, a temperature controller, and an automatically-introducing device, 3447 g of alcohol having 12 carbon atoms (Kao Corporation, product name: Kalcol 2098), 1341g of alcohol having 14 carbon atoms (Kao Corporation, product name: Kalcol 4098), and 6.8 g of KOH were dehydrated for 30 minutes at 110°C under 1.3 kPa. Then, the inside of the autoclave was substituted with nitrogen and elevated to 120°C. 575 g of propylene oxide was added to the mixture. Addition reaction and aging were conducted at 120°C. It was then heated to 145°C. To the mixture was added 1625g of ethylene oxide. Addition reaction and aging were conducted at 145°C. It was then cooled to 80°C. Unreacted ethylene oxide was removed from the mixture at 4.0 kPa (abs). Then, 7.3 g of acetic acid was added into the autoclave. The mixture is stirred for 30 minutes at 80°C and extracted to obtain an alkoxylate having 0.4 mol of the number average addition mole number of propylene oxide and 1.5 mol of the number average addition mole number of ethylene oxide.

The resultant alkoxylate was sulfated with an SO₃ gas in a film reactor for sulfating. To the sulfated product was added an aqueous solution of sodium hydroxide containing ethanol to obtain a surfactant composition containing a polyoxyalkylene alkyl ether sulfate ester salt [component (a)], ethanol [component (b)], and sodium hydroxide [component (c)] at concentrations shown in Table 1. The aqueous solution of sodium hydroxide containing ethanol was used in such amount as that concentrations of ethanol and sodium hydroxide in the surfactant composition were as shown in Table 1. Components analysis of the surfactant composition was performed as described below. The surfactant composition was also evaluated for pH, viscosity, odor and color as described below. Results are shown in Table 1.

### <Method of composition analysis>

An amount of a polyoxyalkylene alkyl ether sulfate ester salt [component (a)] was determined by the Epton method. An amount of ethanol [component (b)] was determined by gas chromatography. An amount of sodium hydroxide [component (c)] was determined by neutralization titration with HCl.

### <pH>

A pH was measured with a pH meter (Horiba Ltd., pH meter D-51) calibrated with a pH standard solution. A surfactant composition or a diluted solution thereof was set to 20°C. A glass pH electrode of the pH meter was dipped therein and left until a constant value was displayed on the pH meter. The value was red.

### <Viscosity>

A surfactant composition was allowed to stand for one hour in a thermostat tank set to a measurement temperature, and measured with a digital viscometer (Tokimec, Inc., model DVL-B II). A viscosity was measured at 20°C.

### <Odor>

A surfactant composition was allowed to stand for 30 days at 50°C in a thermostat tank that can keep the inside temperature thereof at a constant level. The surfactant composition was evaluated just after preparation and after 30 days storage by special researchers working for 10 years or more in a flagrance development division according to the following criteria.
○: strength and kind of odor is the same as that just after preparation.
×: odor is stronger than or differs from that just after preparation.

### <Color>

A surfactant composition was allowed to stand for 15 days at 50°C in a thermostat tank that can keep the inside temperature thereof at a constant level. The surfactant composition was measured for 10% Klett number just after preparation, after standing for 5 days and after standing for 15 days according to the following procedure. The lower the Klett number is, the less the color is.

### *Measurement of 10% Klett number

A surfactant composition was taken in such amount as that containing 10 g of component (a), and added with water to obtain a sample solution of the total volume of 100 mL in a uniform state. The sample solution was adjusted to pH=7.0±0.1 with phosphoric acid. The adjusted solution was placed in a10 mm glass cell, and measured for absorbance at a wave number of 420 nm with a spectrophotometer using water as a reference substance. A measured value was multiplied by 1000 to determine a value of 10% Klett number.

**Table I**

| | | | Example | | | | | Comparative example | |
|---|---|---|---|---|---|---|---|---|---|
| | | | 1 | 2 | 3 | 4 | 5 | 1 | 2 |
| Surfactant composition (% by mass) | (a) | Polyoxyalkylene alkyl ether sulfate ester salt (PO=0.4 mol/EO=1.5mol, block addition) | 54 | 54 | 54 | 54 | 54 | 54 | 54 |
| | (b) | Ethanol | 15 | 15 | 15 | 15 | 15 | 15 | 15 |
| | (c) | NaOH | 0.05 | 0.1 | 0.3 | 0.002 | 0.005 | less than 0.001 | 0.6 |
| | Water | | balance | balance | balance | balance | balance | balance | balance |
| | Total | | 100 | 100 | 100 | 100 | 100 | 100 | 100 |
| pH (20°C) | | | 13.1 | 13.3 | 13.5 | 11.7 | 12.3 | 8.5 | 13.7 |
| Viscosity (mPa·s/20°C) | | | 75 | 75 | 75 | 81 | 81 | 75 | 75 |
| odor (50°C/after 30 days) | | | ○ | ○ | ○ | ○ | ○ | × | ○ |
| Color (10% Klett number) | | Just after preparation | 7 | 6 | 6 | 5 | 5 | 6 | 5 |
| | | 50°C/after 5 days | 7 | 8 | 13 | 4 | 4 | 5 | 25 |
| | | 50°C/after 15 days | 6 | 12 | 23 | 3 | 5 | 5 | - |

In Table 1, the polyoxyethylene alkyl ether sulfate ester salt is a compound of formula (1) (wherein PO and EO are added to RO- in this order by block addition; R represents a mixed alkyl groups composed of alkyl groups having 12 carbon atoms and 14 carbon atoms; m represents 0.4; n represents 1.5; and M represents sodium). Comparative Example 2 was, in view of color change after standing for 5 days, not evaluated for color after standing for 15 days.

### Examples 6 to 8 and Comparative Examples 3 to 4

Surfactant compositions containing ingredients as shown in Table 2 were similarly prepared as in Example 1, except that a compound shown in Table 2 was used as the polyoxyalkylene alkyl ether sulfate ester salt [component (a)]. The polyoxyalkylene alkyl ether sulfate ester salt [component (a)] in Table 2 was prepared by adding 1 mol on the number average of ethylene oxide to an alcohol and then 2.5 mol on the number average of propylene oxide in this order to obtain an ethoxylate sulfate according to the process in Example 1 or etc. Surfactant compositions were similarly evaluated. Results are shown in Table 2.

**Table 2**

| | | | Example | | | Comparative example | |
|---|---|---|---|---|---|---|---|
| | | | 6 | 7 | 8 | 3 | 4 |
| Surfactant composition (% by mass) | (a) | Polyoxyalkylene alkyl ether sulfate ester salt (EO=1 mol/PO=2.5 mol, block addition) | 54 | 54 | 54 | 54 | 54 |
| | (b) | Ethanol | 15 | 15 | 15 | 15 | 15 |
| | (c) | NaOH | 0.05 | 0.1 | 0.3 | Less than 0.001 | 0.6 |
| | Water | | Residue | Residue | Residue | Residue | Residue |
| | Total | | 100 | 100 | 100 | 100 | 100 |
| pH(20°C) | | original solution | 13.1 | 13.1 | 13.6 | 8.5 | 13.7 |
| | | Dilluted solution | 12.5 | 12.4 | 12.6 | 8.5 | 12.5 |
| Viscosity (mPa·s/20°C) | | | 115 | 115 | 115 | 115 | 115 |
| odor (50°C/after 30 days) | | | ○ | ○ | ○ | × | × |
| Color (10% Klett number) | | Just after preparation | 5 | 6 | 6 | 6 | 5 |
| | | 50°C/after 5 days | 6 | 7 | 12 | 8 | 21 |
| | | 50°C/after 15 days | 8 | 11 | 15 | 22 | - |

| | | | | | | | |
|---|---|---|---|---|---|---|---|
| * A solution prepared by diluting a surfactant composition with water such that the solution contained 10% by mass of the component (a). | | | | | | | |

In Table 2, the polyoxyethylene alkyl ether sulfate ester salt is a compound of formula (1) (wherein EO and PO are added to RO- in this order by block addition; R represents a mixed alkyl groups composed of alkyl groups having 12 carbon atoms and 14 carbon atoms; m represents 2.5; n represents 1; and M represents sodium). Comparative Example 4 was, in view of color change after standing for 5 days, not evaluated for color after standing for 15 days.

## Claims

1. A surfactant composition having a pH (20°C) of 10 to 13.6, comprising:
(a) 40 to 58% by mass of a compound represented by formula (1);
(b) 13 to 20% by mass of an alcohol having 1 to 6 carbon atoms;
(c) 0.002 to 0.5% by mass of an alkaline agent; and water:
RO-[(PO)ₘ/(EO)ₙ]SO₃M (1)
wherein, R represents a hydrocarbon group having 8 to 24 carbon atoms; PO and EO represent a propyleneoxy group and an ethyleneoxy group, respectively; m and n represent number average addition mole numbers of PO and EO, respectively, and satisfy 0<m<5 and 0<n<20, respectively; and M represents a cation (excluding a hydrogen ion); wherein the addition mode of PO and EO may be block or random.

2. The surfactant composition according to claim 1, wherein the content of water is 45% or less by mass.

3. The surfactant composition according to claim 1 or claim 2, wherein component (a) is a compound derived from an alcohol derived from natural fat-and-oil.

4. The surfactant composition according to any one of claims 1 to 3, wherein R represents an alkyl group having 8 to 16 carbon atoms.

5. The surfactant composition according to any one of claims 1 to 4, wherein R represents an alkyl group having 12 to 14 carbon atoms.

6. The surfactant composition according to any one of claims 1 to 3, wherein R represents a linear alkyl group or an alkenyl group having 8 to 20 carbon atoms.

7. The surfactant composition according to any one of claims 1 to 6, wherein R represents a hydrocarbon group which is derived from a primary alcohol.

8. The surfactant composition according to any one of claims 1 to 7, wherein m is 0.1 to 3.

9. The surfactant composition according to any one of claims 1 to 8, wherein n is 1 to 20.

10. The surfactant composition according to any one of claims 1 to 9, wherein (a) is a compound in which RO- is linked to PO and EO in this order by block addition.

11. The surfactant composition according to any one of claims 1 to 10, wherein (b) is ethanol.

12. The surfactant composition according to any one of claims 1 to 11, which contains component (c), which is an alkaline agent, in an amount of 0.05 to 0.3 % by mass.

13. A method for producing the surfactant composition according to any one of claims 1 to 12, including the following steps (I) to (III):
step (I): adding propylene oxide and ethylene oxide to 1 mole of an alcohol having a hydrocarbon group having 8 to 24 carbon atoms;
step (II): sulfating the alkoxylate from the step (I); and
step (III): adding water containing (b) and (c) to the alkoxylate sulfate from step (II) to neutralize it, and further adjusting the surfactant composition to have a pH (20°C) of 10 to 13.6.

14. Use of the surfactant composition according to any one of claims 1 to 12 as a liquid detergent.

## Patentansprüche

1. Tensid-Zusammensetzung mit einem pH (20°C) von 10 bis 13,6, umfassend:
(a) 40 bis 58 Masse-% einer Verbindung der Formel (1);
(b) 13 bis 20 Masse-% eines Alkohols mit 1 bis 6 Kohlenstoffatomen;
(c) 0,002 bis 0,5 Masse-% eines alkalischen Mittels; und
Wasser;
RO-[(PO)ₘ/(EO)ₙ]SO₃M (1)
worin R eine Kohlenwasserstoffgruppe mit 8 bis 24 Kohlenstoffatomen darstellt; PO und EO eine Propylenoxygruppe bzw. eine Ethylenoxygruppe darstellen; n und m die zahlengemittelten Additionsmolzahlen von PO bzw. EO darstellen und 0 < m < 5 bzw. 0 < n ≤ 20 erfüllen; und M ein Kation (ausgenommen ein Wasserstoffion) darstellt; wobei der Zugabemodus von PO und EO blockweise oder statistisch sein kann.

2. Tensid-Zusammensetzung gemäss Anspruch 1, wobei der Wassergehalt 45 Masse-% oder weniger beträgt.

3. Tensid-Zusammensetzung gemäss Anspruch 1 oder Anspruch 2, wobei die Komponente (a) eine Verbindung ist, die von einem von natürlichem Fett und Öl abgeleiteten Alkohol abgleitet ist.

4. Tensid-Zusammensetzung gemäss irgendeinem der Ansprüche 1 bis 3, worin R eine Alkylgruppe mit 8 bis 16 Kohlenstoffatomen darstellt.

5. Tensid-Zusammensetzung gemäss irgendeinem der Ansprüche 1 bis 4, worin R eine Alkylgruppe mit 12 bis 14 Kohlenstoffatomen darstellt.

6. Tensid-Zusammensetzung gemäss irgendeinem der Ansprüche 1 bis 3, worin R eine geradkettige Alkylgruppe oder eine Alkenylgruppe mit 8 bis 20 Kohlenstoffatomen darstellt.

7. Tensid-Zusammensetzung gemäss irgendeinem der Ansprüche 1 bis 6, worin R eine von einem primären Alkohol abgeleitete Kohlenwasserstoffgruppe darstellt.

8. Tensid-Zusammensetzung gemäss irgendeinem der Ansprüche 1 bis 7, worin m 0,1 bis 3 ist.

9. Tensid-Zusammensetzung gemäss irgendeinem der Ansprüche 1 bis 8, worin n 1 bis 20 ist.

10. Tensid-Zusammensetzung gemäss irgendeinem der Ansprüche 1 bis 9, worin (a) eine Verbindung ist, in der RO- mit PO und EO in dieser Reihenfolge durch Blockaddition verbunden ist.

11. Tensid-Zusammensetzung gemäss irgendeinem der Ansprüche 1 bis 10, worin (b) Ethanol ist.

12. Tensid-Zusammensetzung gemäss irgendeinem der Ansprüche 1 bis 11, die die Komponente (c), die ein alkalisches Mittel ist, in einer Menge von 0,05 bis 0,3 Masse-% enthält.

13. Verfahren zur Herstellung der Tensid-Zusammensetzung gemäss irgendeinem der Ansprüche 1 bis 12, das die nachstehenden Schritte (I) bis (III) einschliesst:
Schritt (I):
Zugabe von Propylenoxid und Ethylenoxid zu 1 mol eines Alkohols, der eine Kohlenwasserstoffgruppe mit 8 bis 24 Kohlenstoffatomen aufweist;
Schritt (II):
Sulfatieren des Alkoxylats aus Schritt (I); und
Schritt (III):
Zugabe von Wasser, das (b) und (c) enthält, zu dem Alkoxylatsulfat aus Schritt (II), um dieses zu neutralisieren, und ferner Einstellen der Tensid-Zusammensetzung auf einen pH (20°C) von 10 bis 13,6.

14. Verwendung der Tensid-Zusammensetzung gemäss irgendeinem der Ansprüche 1 bis 12 als flüssiges Detergens.

## Revendications

1. Composition tensioactive ayant un pH (20°C) de 10 à 13,6, comprenant :
(a) de 40 à 58% en masse d'un composé représenté par la formule (1) ;
(b) de 13 à 20% en masse d'un alcool ayant 1 à 6 atomes de carbone ;
(c) de 0,002 à 0,5% en masse d'un agent alcalin ; et de l'eau :
RO-[(PO)ₘ/(EO)ₙ]SO₃M (1)
où, R représente un groupe d'hydrocarbure ayant 8 à 24 atomes de carbone ; PO et EO représentent un groupe propylèneoxy et un groupe éthylèneoxy, respectivement ; m et n représentent des nombres de moles moyens d'addition en nombre de PO et EO, respectivement, et satisfont 0<m<5 et 0<n≤20, respectivement ; et M représente un cation (à l'exception d'un ion hydrogène) ; où le mode d'addition de PO et EO peut être séquencé ou aléatoire.

2. Composition tensioactive selon la revendication 1, dans laquelle la teneur en eau est de 45% en masse ou moins.

3. Composition tensioactive selon la revendication 1 ou 2, dans laquelle un composant (a) est un composé dérivé d'un alcool dérivé d'huile et de graisses naturelles.

4. Composition tensioactive selon l'une quelconque des revendications 1 à 3, dans laquelle R représente un groupe alkyle ayant 8 à 16 atomes de carbone.

5. Composition tensioactive selon l'une quelconque des revendications 1 à 4, dans laquelle R représente un groupe alkyle ayant 12 à 14 atomes de carbone.

6. Composition tensioactive selon l'une quelconque des revendications 1 à 3, dans laquelle R représente un groupe alkyle linéaire ou un groupe alcényle ayant 8 à 20 atomes de carbone.

7. Composition tensioactive selon l'une quelconque des revendications 1 à 6, dans laquelle R représente un groupe d'hydrocarbure qui est dérivé d'un alcool primaire.

8. Composition tensioactive selon l'une quelconque des revendications 1 à 7, dans laquelle m est de 0,1 à 3.

9. Composition tensioactive selon l'une quelconque des revendications 1 à 8, dans laquelle n est de 1 à 20.

10. Composition tensioactive selon l'une quelconque des revendications 1 à 9, dans laquelle (a) est un composé dans lequel RO- est lié à PO et EO dans cet ordre par addition séquencée.

11. Composition tensioactive selon l'une quelconque des revendications 1 à 10, dans laquelle (b) est de l'éthanol.

12. Composition tensioactive selon l'une quelconque des revendications 1 à 11, qui contient le composant (c), qui est un agent alcalin, en une quantité de 0,05 à 0,3% en masse.

13. Procédé de production de la composition tensioactive selon l'une quelconque des revendications 1 à 12, comportant les étapes (I) à (III) suivantes :
étape (I) : l'ajout d'oxyde de propylène et d'oxyde d'éthylène à 1 mole d'un alcool ayant un groupe d'hydrocarbure ayant 8 à 24 atomes de carbone ;
étape (II) : la sulfatation de l'alcoxylate de l'étape (I) ; et
étape (III) : l'ajout de l'eau contenant (b) et (c) au sulfate d'alcoxylate de l'étape (II) pour le neutraliser, et l'ajustement en outre de la composition tensioactive de manière à avoir un pH (20°C) qui se situe dans la plage de 10 à 13,6.

14. Utilisation de la composition tensioactive selon l'une quelconque des revendications 1 à 12 en tant que détergent liquide.
